# FASCICULE DE BREVET EUROPEEN

(11) **EP 0 691 978 B1**
(45) Date de publication et mention de la délivrance du brevet: **08.10.1997**
(21) Numéro de dépôt: 94911996.0
(22) Date de dépôt: 30.03.1994
(51) Int. Cl.: C07H 21/00, C12Q 1/68, C25B 3/10, C25B 11/04, C08G 61/12

(54) **COPOLYMERE NUCLEOTIDE(S)/POLYMERE CONDUCTEUR ELECTRONIQUE, SON PROCEDE DE PREPARATION ET SON UTILISATION**
NUKLEOTIDE/ELEKTRONISCHE LEITFÄHIGE POLYMER COPOLYMERISAT, VERFAHREN ZU SEINER HERSTELLUNG UND SEINE VERWENDUNG
ELECTRONICALLY CONDUCTIVE POLYMER/NUCLEOTIDE COPOLYMER, PREPARATION METHOD THEREFOR AND USE THEREOF

(30) Priorité: 31.03.1993 FR 9303732
(43) Date de publication de la demande: 17.01.1996
(73) Titulaire: CIS BIO INTERNATIONAL, 91400 Saclay (FR)
(72) Inventeur: TEOULE, Robert, F-38000 Grenoble (FR); ROGET, André, F-38120 Saint-Egrève (FR); LIVACHE, Thierry, F-38000 Grenoble (FR); BARTHET, Christelle, F-38100 Grenoble (FR); BIDAN, Gérard, F-38100 Grenoble (FR)
(74) Mandataire: Vialle-Presles, Marie José
(86) Numéro de dépôt international: FR9400354
(87) Numéro de publication internationale: WO9422889

(56) Documents cités:
- EP-A- 0 314 009
- WO-A-91/08307
- WO-A-92/07882
- EP-A- 0 314 009
- WO-A-91/08307
- WO-A-92/07882

## Description

La présente invention est relative à la fixation d'acides nucléiques sur un polymère conducteur électronique (PCE).

Dans un grand nombre de techniques couramment utilisées en biologie, par exemple la synthèse ou l'hybridation d'acides nucléiques, des oligonucléotides sont fixés de façon covalente par leur extrémité à un support solide. Différents supports ont été utilisés dans ce but : le papier, le nylon, le verre, la silice, le polystyrène, le polyacrylamide, etc...

A l'heure actuelle, de nombreuses équipes cherchent à obtenir des supports portant un grand nombre d'oligonucléotides de séquences différentes, disposées selon un arrangement préétabli, afin de réaliser simultanément différentes réactions (hybridation sur support par exemple).

C'est ainsi que, par exemple, cette approche a été proposée pour faciliter le séquençage des acides nucléiques.

Des oligonucléotides différents disposés en rangées et colonnes sur des microsurfaces (matrices d'oligonucléotides sur support) ont été proposés pour séquencer les acides nucléiques [LYSOV et al, Proc. USSR Acad. Sci., 303, 1508- 1511, (1988) ; KHRAPKO et al., FEBS Lett. 256, 118-122, (1989) ; KHRAPKO et al., DNA Séquence, vol 1, 375-388 (1991) ; BAINS & SMITH, J.Theor.Biol. 135, 303-307, (1988) ; CHURCH & KIEFFER-HIGGINS, Science 240, 185-188 (1988) ; SOUTHERN, Demande PCT WO89/10977 (1989)]. La méthode est basée sur l'hybridation de chaînes d'ADN ou d'ARN cibles sur un ensemble d'oligonucléotides. Théoriquement, la présence ou l'absence d'une séquence dans l'acide nucléique cible peut être déterminée par l'hybridation observée sur les micro-surfaces dans des conditions de stringence déterminées.

En ce qui concerne la synthèse *in situ* de polynucléotides ou de polypeptides, FODOR et al. [Science, 251, 767-773 (1991)], en combinant les méthodes de la synthèse chimique en phase solide, les groupements photolabiles et la photolithographie, ont réussi à synthétiser 1024 peptides sur une matrice de points (carrés de 100 µm de côté). Ces peptides ont été obtenus par synthèses simultanées et parallèles, en utilisant des masques de photolithographie et des groupements protecteurs photolabiles des synthons peptidiques. Un dinucléotide dCpT a été préparé in situ, en utilisant la thymidine protégée en 5' par un groupement protecteur photolabile (5'-nitrovératryl thymidine) . La lumière était dirigée par un masque de photolithographie et un dépôt en damier de 100 µm de côté a été obtenu.

MASKOS & SOUTHERN (Nucleic Acids Res. 1992, 20, 1675-1678) ont réalisé, sous microscope, la synthèse in situ de quatre oligonucléotides différents sur une lame de verre.

Jusqu'à présent, les techniques utilisées pour le dépôt adressé d'oligonucléotides font appel, soit au dépôt manuel (qui n'est pas utilisable à l'échelon industriel), soit aux techniques de photolithographie, qui nécessitent l'utilisation de "masques" et en outre, sont difficilement applicables avec les acides nucléiques, qui sont photolabiles.

La présente Invention s'est fixé pour but l'obtention de nouveaux supports et de nouveaux procédés de fixation d'oligonucléotides, qui ne présentent pas les inconvénients des procédés proposés dans l'art antérieur.

Dans ce but, les Inventeurs ont eu l'idée d'utiliser comme support de fixation des polymères conducteurs électroniques.

La Demande EP 314 009 décrit des monomères de polymère conducteur électronique, constitués par des dérivés de 2,5-di(2-thiényl)pyrrole, substitués en position 3 du cycle pyrrole par diverses fonctions qui permettent la fixation, par liaison covalente, de peptides et de protéines sur le polymère conducteur électronique résultant de la polymérisation de ces dérivés. Ce document ne donne aucune indication quant à la possibilité de fixer des oligonucléotides sur un polymère conducteur électronique.

Les Inventeurs sont maintenant parvenus à fixer par liaison covalente, et de façon stable, des nucléotides et des oligonucléotides sur un polymère conducteur électronique, et à obtenir de la sorte de nouveaux copolymères.

La présente invention a pour objet un copolymère, caractérisé en ce qu'il répond à la formule générale (I) suivante : 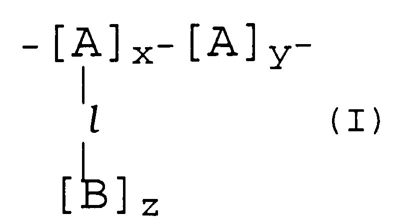 dans laquelle l'unité A représente un monomère d'un polymère conducteur électronique, l'unité B représente un nucléotide, un oligonucléotide ou un de leurs analogues, x, y et z représentent des nombres entiers égaux ou supérieurs à 1, ou y peut être égal à 0, et *l* représente une liaison covalente, ou un bras espaceur.

A titre d'exemple non limitatif de polymères conducteurs électroniques dont A représente un monomère on citera le polyacétylène, la polyazine, le poly(p-phénylène), le poly(p-phénylène vinylène), le polypyrène, le polypyrrole, le polythiophène, le polyfuranne, le polysélénophène, la polypyridazine, le polycarbazole, la polyaniline, etc...

Avantageusement, A est une unité pyrrole.

Dans le cadre de l'exposé de la présente Invention, on entend par analogue de nucléotide, tout nucléotide modifié, tels que ceux décrits par exemple par UHLMANN, [Chemical Review, 90:4, 543-584 (1990)].

Lorsque l'unité B est un nucléotide, il peut s'agir non seulement d'un de ceux qui entrent habituellement dans la composition des oligonucléotides naturels, mais également leurs analogues ou dérivés utilisés en laboratoire.

Il peut s'agir par exemple :
* d'analogues de nucléotides entrant dans la composition d'oligonucléotides synthétiques ;
* de dérivés de nucléotides portant des fonctions protégées qui sont couramment utilisés pour la synthèse des acides nucléiques ; B_{z} peut dans ce cas constituer un intermédiaire de synthèse d'un oligonucléotide.

B_{z} pourra aussi être un composé non naturel pouvant s'hybrider avec les acides nucléiques, tels que ceux décrits par UHLMANN (publication précitée).

Les unités B entrant dans la constitution de B_{z} peuvent être identiques ou différents, et B_{z} peut constituer un homopolymère ou un hétéropolymère ; dans ce dernier cas, les unités B peuvent s'enchaîner selon une séquence quelconque, prédéterminée ou non.

Selon un mode de réalisation préféré de la présente invention le rapport x/y est compris entre 1/5 et 1/100.000

Selon encore un autre mode de réalisation préféré de la présente invention, *l* représente un bras espaceur répondant à l'une des formules suivantes :

-R₁-[(CH₂)ₙ-R₂]ₓ-[(CH₂)ₘ-R₃]_{y}-(CH₂)ₚ-

dans laquelle :
- n est un nombre entier de 1 à 10 ;
- m est égal à 0 ou est un nombre entier de 1 à 10 ;
- p est égal à 0 ou est un nombre entier de 1 à 10 ;
- x est égal à 0 ou est un nombre entier de 1 à 8 ;
- y est égal à 0 ou est un nombre entier de 1 à 8 ;
- R₁, R₂ et R₃ qui peuvent être identiques ou différents représentent :
   CH2; O; S; NR'; CO; CH=CH ; NR'CO; CONR'; NHSO₂; 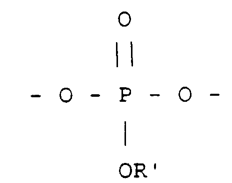 où R' représente un atome d'hydrogène ou une chaîne alkyle en C₁ à C₁₂.

La présente invention a pour objet l'utilisation d'un polymère conducteur électronique comme support pour la fixation par liaison covalente, d'au moins un nucléotide.

La présente Invention a également pour objet un procedé de préparation d'un copolymère de formule générale (I).

Selon une première variante, ce procédé est caractérisé en ce qu'il comprend au moins les étapes suivantes
- une première étape, au cours de laquelle on procède à la préparation d'un copolymère de formule générale (II) :

   - [A*]ₓ-[A]_{y} - (II)

   dans laquelle A, x et y sont tels que définis précédemment, et A* représente A fonctionnalisé.
- une deuxième étape au cours de laquelle on procède à la fixation, sur le polymère de formule (II) d'au moins un groupe de formule générale (III) :

   *l**-[B]_{z} (III)

   dans laquelle B et z sont tels que définis précédemment et *l** est un bras activé capable de se lier à A*.

On entend par "fonctionnalisé" et "activé" au sens de la présente Invention, le résultat de toute modification chimique ayant pour but de pourvoir A et *l* de fonctions chimiques capables de réagir entre elles pour former une liaison covalente.

Selon une autre variante, le procédé de préparation d'un copolymère de formule générale (I) est caractérisé en ce qu'il comprend au moins les étapes suivantes :
- une étape a) au cours de laquelle on procède à la préparation du composé de formule générale (IV) : 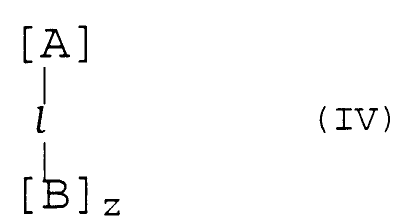 dans lequel A, B, z, et *l* sont tels que définis ci-dessus,
- une étape b) au cours de laquelle on procède à la copolymérisation du composé (IV) avec le monomère A.

Avantageusement, au moins une étape de l'une ou l'autre des variantes du procédé conforme à l'invention fait intervenir au moins une réaction électrochimique. Cette copolymérisation électrochimique est avantageusement effectuée en surface d'une électrode ; en fin de réaction on obtient de la sorte une électrode dont la surface est constituée par un copolymère conforme à l'invention.

Par exemple, pour la mise en oeuvre de la première variante du procédé conforme à l'invention, l'étape de préparation du copolymère de formule générale (II) et/ou l'étape de fixation du groupe de formule générale (III) peuvent être effectuées par réaction électrochimique ; dans la seconde variante, l'étape b) est avantageusement effectuée par copolymérisation électrochimique du composé (IV) avec les monomères A.

La copolymérisation électrochimique est par exemple effectuée par voltampérométrie cyclique, en soumettant le mélange [(IV) : A] à des variations de potentiel électrique suffisantes pour provoquer la polymérisation par une oxydation et une réduction successives ; le polymère formé étant conducteur, le cycle oxydation-réduction peut être répété plusieurs fois.

Les méthodes de polymérisation électrochimique généralement utilisées pour la préparation des PCE, telles que la polymérisation à courant (chronopotentiométrie) ou à potentiel (chronoampérométrie) imposés sont également applicables à la préparation des copolymères conformes à l'Invention.

La qualité du dépôt peut être contrôlée par le choix des conditions expérimentales : le rapport oligonucléotide-pyrrole/pyrrole, la température du bain, la nature du solvant, la méthode électrochimique utilisée (voltampèrométrie cyclique, chronoampèrométrie, chronopotentiométrie). Le copolymère obtenu peut de la sorte présenter des qualités de porosité et d'accessibilité différentes selon l'usage ultérieur souhaité, et la quantité d'oligonucléotide fixé peut être modulée.

Avantageusement, dans le cadre de la mise en oeuvre du procédé conforme à l'invention, les réactions électrochimiques sont effectuées à la surface d'une électrode. L'électrode permet en effet de contrôler, par mesure du courant délivré au cours de la réaction, l'évolution de la réaction de polymérisation (par exemple l'épaisseur du polymère formé), ou de réactions ultérieures effectuées sur le copolymère.

Selon un mode de réalisation préféré du procédé conforme à l'invention dans l'une ou l'autre de ses variantes, il comprend en outre l'élongation de l'oligonucléotide B_{z}, en plusieurs étapes successives, chacune de ces étapes étant constituée par la fixation d'un ou plusieurs unités B.

L'élongation de l'oligonucléotide B_{z} s'effectue à la surface du support par assemblage de monomères protégés, à partir d'au moins un nucléotide ou oligonucléotide fixé en surface du polymère conducteur électronique.

Les méthodes classiques de synthèse par voie chimique des acides nucléiques sont utilisables dans la mise en oeuvre de ce mode de réalisation.

Les supports conformes à l'Invention permettent en outre de réaliser l'élongation de l'oligonucléotide par voie électrochimique, en utilisant des variations de potentiel de l'électrode pour effectuer les réactions de protection, de déprotection et de condensation de la chaine polymérique en croissance.

La présente invention a également pour objet une électrode, caractérisée en ce que sa surface est constituée par un revêtement comprenant un copolymère de formule (I) conforme à l'Invention.

Une telle électrode peut être obtenue, par exemple, en déposant une couche d'un copolymère de formule (I) à la surface d'une électrode de platine, d'or, de chrome ou de titane recouvert d'or, ou de carbone vitreux, etc ...

Avantageusement, on peut associer plusieurs électrodes portant éventuellement des copolymères de nature différente. On obtient ainsi un dispositif utilisable pour la mise en oeuvre de réactions de synthèse, et/ou de réactions d'hybridation d'acides nucléiques.

Un mode de réalisation particulièrement avantageux d'un dispositif conforme à l'Invention consiste à associer plusieurs électrodes dont deux au moins portent un groupe B_{z} différent. Il peut s'agir par exemple d'un ensemble d'électrodes dont chacune porte un nucléotide (ou analogue) différent, ou d'un ensemble d'électrodes dont chacune porte un oligonucléotide de séquence différente.

Dans la mesure où il est possible de limiter les réactions électrochimiques aboutissant à la fixation de l'oligonucléotide à une très petite surface, un dispositif conforme à l'invention peut être constitué par une pluralité de microsurfaces de PCE portées par des microélectrodes distribuées sur un support (microchip PCE). De la sorte, des oligonucléotides B_{z} qui peuvent, si on le souhaite, être tous différents, peuvent être fixés de façon adressée et ordonnée sur ces microélectrodes.

Le "microchip PCE" est en particulier utilisable pour le séquençage des acides nucléiques et le diagnostic.

A titre d'exemple non limitatif illustrant ce qui précède, un copolymère [polypyrrole portant des oligonucléotides/polypyrrole] conforme à la présente invention, peut être obtenu :
1) Par réaction chimique d'un nucléoside, d'un nucléotide, d'un oligonucléotide, ou d'un de leurs analogues sur un polypyrrole fonctionnalisé. Par exemple, il est possible d'effectuer la condensation de l'aminoéthylpyrrole avec un oligonucléotide portant à une extrémité un phosphate libre ou un carboxyle activé.
2) Par copolymérisation chimique ou électrochimique du pyrrole avec le produit de condensation d'un nucléoside, d'un nucléotide, d'un oligonucléotide, ou d'un de leurs analogues avec le pyrrole. Par exemple on peut procéder à la copolymérisation électrochimique du pyrrole avec un oligonucléotide ayant un bras espaceur portant un pyrrole à son extrémité. L'épaisseur de la couche du copolymère obtenue sur une surface de platine à laquelle elle adhère fortement est de 0,1 µm à quelques µm, et elle peut être réalisée sur une surface de 100 µm² par exemple. Aucune réaction parasite de dégradation de l'oligonucléotide n'a pu être mise en évidence.
3) par préparation d'un polymère conducteur électronique portant des fonctions chimiques protégées. Ces fonctions sont déprotégées localement et sélectivement pour permettre leur couplage avec un nucléoside, un nucléotide ou un oligonucléotide. Par exemple, il est possible de réaliser la préparation de monométhoxytrityl aminoéthyl pyrrole/polypyrrole, et de le déprotéger localement soit en milieu acide, soit en appliquant un potentiel. La fonction amine libérée peut ensuite réagir avec un nucléoside, un nucléotide ou un oligonucléotide portant par exemple un phosphate ou un carboxyle activés.
4) par synthèse simultanée dirigée spatialement de différents oligonucléotides.

La synthèse d'un oligonucléotide s'effectue en un point du support par assemblage de nucléotides protégés, à partir d'un nucléoside accessible en surface du copolymère. Les nucléotides protégés peuvent être des nucléosides phosphoramidites, des nucléosides phosphonates, des nucléosides phosphotriesters. Localement, la synthèse est effectuée à la manière dont est réalisée la synthèse d'un oligonucléotide sur support de silice dans un synthétiseur. Mais la différence est que la synthèse de tout l'ensemble des oligonucléotides est réalisée simultanément, en réalisant électrochimiquement des opérations de déprotection ou de condensation sélectives sur une très petite surface, ce qui permet de masquer les oligonucléotides qui ne doivent pas réagir. Ceci permet de réaliser en parallèle la synthèse de différents oligonucléotides.

Bien entendu, les procédés brièvement exposés ci dessus pour illustrer la synthèse de copolymères [pyrrole/oligonucléotides-pyrrole] sont également appliquables à des analogues polynucléotidiques, par exemple des analogues de la chaîne sucre-phosphate tels que mono- ou dithiophosphates, méthylphosphonates, phosphotriesters et des analogues non-ioniques non phosphorylés tels que formacétals, carbamates, sulfoxydes.

Les copolymères conformes à l'invention présentent une bonne stabilité aux contraintes mécaniques, à l'humididité, à la dessication, à la chaleur, aux bases, et sont donc compatibles avec un grand nombre de réactions, ce qui autorise une large variété d'utilisations.

Les inventeurs ont procédé à l'hybridation sélective d'oligonucléotides à des oligonucléotides complémentaires fixés sur support de polypyrrole, et ont constaté que l'utilisation de ce support confère les avantages suivants :
- Le copolymère conforme à l'invention est poreux, ce qui confère aux oligonucléotides fixés sur le support une bonne accessibilité pour l'hybridation avec des acides nucléiques de séquence complémentaire. Cette accessibilité est mise en évidence par l'observation d'une hybridation proportionnelle à l'épaisseur de la couche de copolymère. Un oligonucléotide complémentaire se trouvant dans le milieu d'hybridation s'hybride trois fois plus sur une couche de copolymère pyrrole/oligonucléotide-pyrrole trois fois plus épaisse (et donc renfermant trois fois plus d'oligonucléotide lié au support). La cinétique d'hybridation est voisine de celle qu'on observe avec les supports d'hybridation conventionnels. Il faut noter que dans les mêmes conditions, un oligonucléotide de séquence non complémentaire ne se fixe pas sur le support.
- Les Inventeurs ont également vérifié que l'hybridation est réversible et que tout oligonucléotide hybridé peut être relargué par chauffage, ou par traitement avec de la soude diluée, sans dommage pour le polypyrrole et l'oligonucléotide fixé.
- Comme il a été indiqué précédemment, une copolymérisation adressée est réalisable sur des surfaces d'électrodes extrêment petites. Ceci permet de réaliser une matrice de points miniaturisée parfaitement ordonnée sur un support, chacun de ces points portant un oligonucléotide de nature parfaitement définie. Les chaînes nucléiques cibles portant une séquence complémentaire à la chaîne fixée sur le support s'hybrident sélectivement. Il en résulte une densité locale d'acides nucléiques cibles extrêment élevée, ce qui rend leur détection plus aisée, voire même dans certains cas supprime la nécessité d'une amplification préalable à la détection. La détection de l'hybridation peut en particulier être faite par le biais de l'électrode qui a servi à préparer le copolymère, et qui peut servir ensuite pour la mesure des phénomènes d'association ou de dissociation qui se produiront à sa surface. L'hybridation d'un acide nucléique complémentaire peut par exemple être suivie *in situ* par mesure électrique sur l'électrode qui supporte le polymère conducteur électronique, soit par mesure directe, soit en marquant l'oligonucléotide cible par une molécule électroactive telle qu'une phénothiazine ou une quinone par exemple.

Il va de soi que les méthodes traditionnelles de détection des séquences cibles d'acides nucléiques sont également applicables.

Les inventeurs ont en outre réussi à synthétiser des oligonucléotides directement sur le copolymère conforme à l'Invention par déprotection électrochimique in situ.

De manière générale, l'assemblage d'un nucléotide sur une chaîne polynucléotidique en croissance sur un support fait appel à une série de réactions qui mettent en jeu des groupements protecteurs pour diriger la réaction sur une fonction donnée et l'empêcher sur une autre. Les Inventeurs ont mis cette propriété à profit pour orienter la réaction d'assemblage sur les surfaces correspondant aux oligonucléotides choisis où l'on veut insérer un nucléotide.

Conformément à l'Invention, le groupement protecteur de la chaîne en croissance de l'oligonucléotide peut être éliminé localement par une réaction électrochimique, ce qui permet d'ajouter un nucléotide à l'emplacement choisi.

Des avantages complémentaires découlent de cette possibilité d'effectuer, sur le support conforme à l'invention, une synthèse oligonucléotidique in situ. En effet, dans ce cas, il est possible de synthétiser in situ et en parallèle l'ensemble des oligonucléotides qui vont être disposés sur la matrice de points, au lieu de synthétiser indépendamment des oligonucléotides portant un bras pyrrole, puis d'effectuer des copolymérisations successives. Ceci permet d'envisager la réalisation industrielle de matrices de plusieurs milliers de microsurfaces.

La présente invention sera mieux comprise à l'aide du complément de description qui va suivre, qui se réfère à des exemples de préparation et d'utilisation de copolymères conformes à l'invention.

### PREPARATION D'UN SUPPORT POLYPYRROLE PAR COPOLYMERISATION DE PYRROLE ET D'UN OLIGONUCLEOTIDE PORTANT UN GROUPE PYRROLE ; PROPRIETES DE CE SUPPORT

### EXEMPLE N° 1 : SYNTHESE DES OLIGONUCLEOTIDES MODIFIES

### I. - PREPARATION D'UN AMIDITE DE NUCLEOSIDE PYRROLE :

### 1ère METHODE

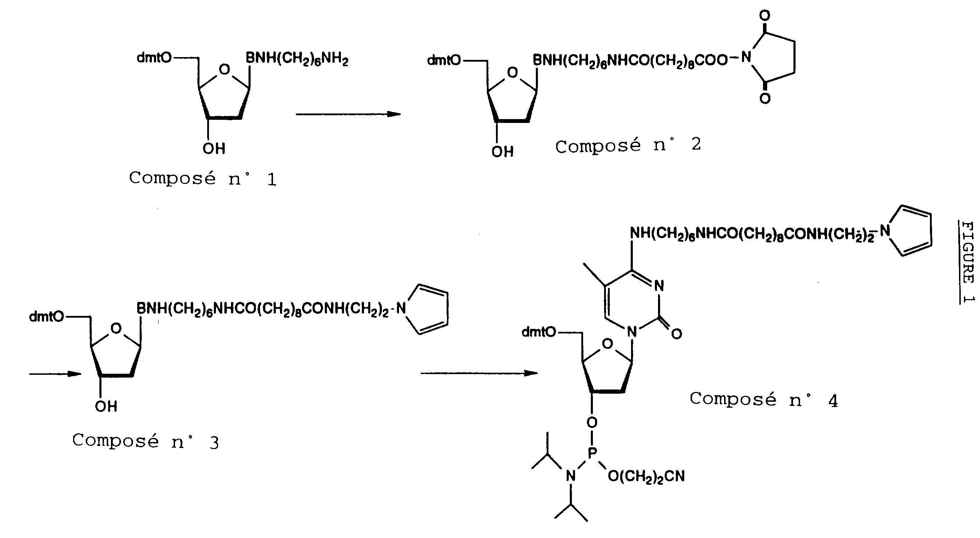

Le schéma réactionnel global de cette synthèse est illustré à la figure 1.

### - Préparation du composé n° 1 (figure 1)

Ce composé peut être obtenu par réaction d'une diamine sur la diméthoxytrityl thiothymidine ou sur la diméthoxytrityl thiodésoxyuridine suivant les méthodes décrites par ROGET et al., [Nucleic Acids Res. 17, 7643-7651 (1989)].

### - Préparation du composé n°2 (figure 1)

Le composé n°1 (2 g ; 3,1 mmoles) est séché par coévaporation dans l'acétonitrile anhydre et redissous dans 20 ml de dichlorométhane. On ajoute 2 eq de dissuccinimidyl sébacoate (2,45 g ; 6,2 mmoles). La réaction est laissée 3 heures à température ambiante. Le produit obtenu est séparé sur colonne de silice (gradient de 0 à 10% de méthanol dans le chloroforme) ou précipité dans l'hexane (R = 60%). Il peut aussi être utilisé tel quel pour la synthèse du composé n° 3.

### - Préparation du composé n° 3 (figure 1)

Ce produit est préparé par ajout de l'aminoéthylpyrrole (1,36 g ; 12,4 mmoles) au mélange réactionnel précédent ou de 220 mg d'aminoéthyl pyrrole (2 mmoles) au composé n° 2 obtenu après purification. Le pH est amené à 8-8,5 par ajout d'une amine tertiaire (triéthylamine). La réaction est laissée 2 heures et on ajoute 250 ml de chloroforme. La solution organique obtenue est lavée par 2 fois 100 ml de NaHCO3 O,5 M, et 100 ml d'eau distillée, puis séchée sur sulfate de sodium. Le produit est séparé sur colonne de silice avec du méthanol dans le chloroforme (0 à 10%). Après évaporation du solvant, le composé n° 3 est repris par 10 ml d'éthanol et précipité dans 400 ml d'éther éthylique (R = 60%).

### - Préparation du composé n° 4 (figure 1)

Le composé n°3 (100 mg ; 0,11 mmoles) et du tétrazolate de diisopropylammonium (9 mg ; 0,5 eq) sont séchés par coévaporation dans un mélange de dichlorométhane (2 ml) et d'acétonitrile (3 ml) anhydres. Le résidu est repris par 2,5 ml de dichlorométhane stabilisé à l'amylène. De la bis-diisopropylaminocyanoéthoxyphosphine (39 µl ; 1,2 eq) est ajoutée à travers un septum. Au bout de 2 heures de réaction, on ajoute 20 ml de dichlorométhane anhydre. La solution obtenue est lavée 2 fois par 25 ml de NaHCO3 saturé puis 25 ml d'eau distillée. La phase organique est séchée sur sulfate de sodium et évaporée à sec. Le phosphoramidite obtenu est repris par 2 ml de dichlorométhane, précipité dans 100 ml d'hexane et séché une nuit au dessicateur. Le composé n°4 est obtenu avec un rendement de 85%. Il est conservé sous argon à -20°C à l'abri de l'humidité.

### 2° METHODE :

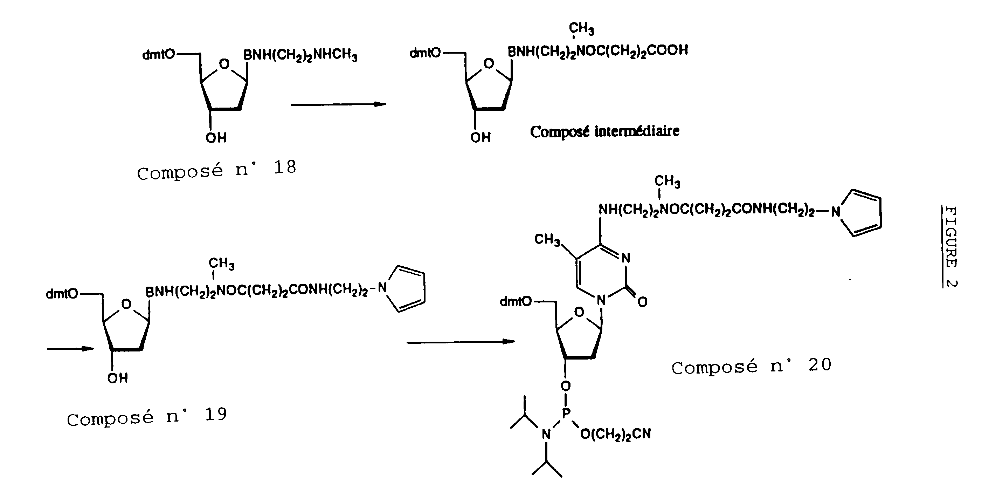

Les étapes de ce procédé sont illustrées à la figure 2.

### MODE OPERATOIRE :

### Préparation du composé n° 18 (figure 2)

Ce composé peut être préparé par réaction d'une diamine sur la diméthoxytrityl thiothymidine ou sur la diméthoxytrityl thiodésoxyuridine suivant les méthodes décrites par ROGET et al. [Nucleic Acids Res. ; 17, 7643-7651 (1989)].

### Préparation du composé n° 19 (figure 2)

Le composé n°18 (4 g ; 6.60 mmoles) est séché par coévaporation dans la pyridine anhydre et redissous dans 4 ml de pyridine anhydre et 40 ml de THF (tétrahydrofurane) anhydre.

On ajoute 1.2 eq d'anhydride succinique (800 mg ; 8 mmoles), et on laisse réagir pendant 1 h. On évapore les solvants puis, afin d'éliminer la pyridine, on coévapore au toluène. On ajoute 1.5 eq d'aminoéthyl pyrrole (1,1 ml ; 9,9 mmoles). On coévapore au THF. On ajoute 30 ml de THF et 2 eq de DCC (dicyclohexylcarbodiimide) (2,70 g ; 13.2 mmole) préalablement dissoute dans 30ml de THF. On laisse la réaction la nuit. Le précipité est éliminé par filtration et lavé par du dichlorométhane jusqu'à ce qu'il soit blanc. Le filtrat est évaporé à sec et repris par 250 ml de dichlorométhane. La solution organique obtenue est lavée par 2 x 250 ml de NaHCO₃ saturé puis 250 ml d'eau distillée. La phase organique est séchée sur sulfate de sodium et évaporée à sec. Le produit (composé n° 19) est purifié par chromatographie sur colonne de silice. Il est élué par CH₂CL₂/MeOH/TEA : 97,5/2,5/1. R = 67 %.

### Préparation du composé n° 20 (figure 2)

Le composé n° 19 (600 mg ; 0.75 mmoles), et du tétrazolate de diisopropylammonium (63 mg ; 0.37 mmoles) sont séchés par coévaporation dans du dichlorométhane/acétonitrile: 2,5 ml/2,5 ml, puis repris par 5 ml de dichlorométhane. La bis-diisopropylaminocyanoéthoxyphosphine (280 µl ; 0.9 mmoles) est ajoutée à travers un septum. Au bout de 2 heures de réaction on ajoute 20 ml de dichlorométhane. La solution organique obtenue est lavée par 2 x 25 ml de NaHCO₃ saturé puis 25 ml de NaCl saturé. La phase organique est séchée sur Na₂SO₄ et évaporée à sec. Le résidu de l'évaporation est repris par 5 ml de dichlorométhane. Le produit (composé n° 20) est obtenu par précipitation dans l'hexane, avec un rendement de 78 %. Après séchage une nuit dans un dessicateur sous vide, il est conservé sous argon à -20°C à l'abri de l'humidité.

### II. - PREPARATION D'UN NUCLEOSIDE PYRROLE

### - Préparation du composé n° 5

Le composé n°4 (68 mg ; 0,06 mmoles) est redissous dans 300 µl d'acétonitrile anhydre (solution 0,2 M). Ce produit est utilisé pour préparer un oligonucléotide (Oligo-1-pyr) de séquence Pyr-TGT ACC TGA ATC GTC CGC CAT, dans laquelle Pyr représente le dérivé de nucléotide correspondant au composé n°4. La préparation de cet oligonucléotide est réalisée sur un synthétiseur automatique d'ADN (Applied Biosystems 381A) suivant les procédures décrites par le fabricant. Le composé n° 4 de l'invention est soumis au même cycle de synthèse que les phosphoramidites normaux (A C G T). Seuls la concentration (0,2 M au lieu de 0,1 M) et le temps de réaction (30 secondes au lieu de 15 secondes) sont augmentés pour le composé n° 4.

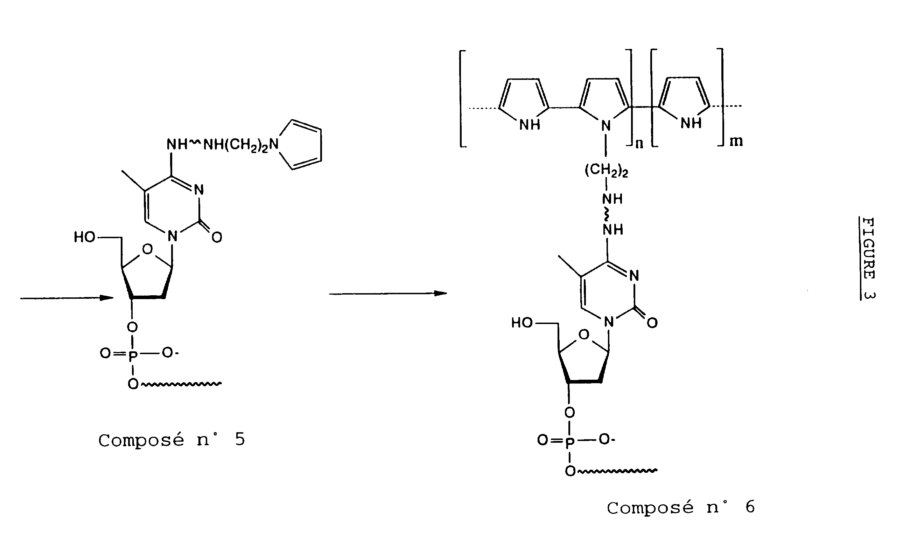

Après synthèse, l'oligonucléotide-pyrrole est détritylé sur le support, par action de TCA (acide trichloracétique) à 3%. Il est coupé du support par 4 x 500 µl de NH₄OH à 28%. Le chauffage de cette solution pendant 16 heures à 60°C permet d'éliminer les groupements protecteurs. Le composé n°5 (représenté figure 3) est obtenu par chromatographie en phase inverse en utilisant un gradient de 10 à 50% d'acétonitrile dans l'acétate de triéthylammonium (25 mM, pH 7).

Le composé n 20 peut être utilisé de la même façon que le composé n° 4.

### EXEMPLE 2 : PREPARATION DU SUPPORT PCE-POLYNUCLEOTIDE PAR COPOLYMERISATION ELECTRONIQUE (composé n° 6, figure 3)

### A - Principe de la technique

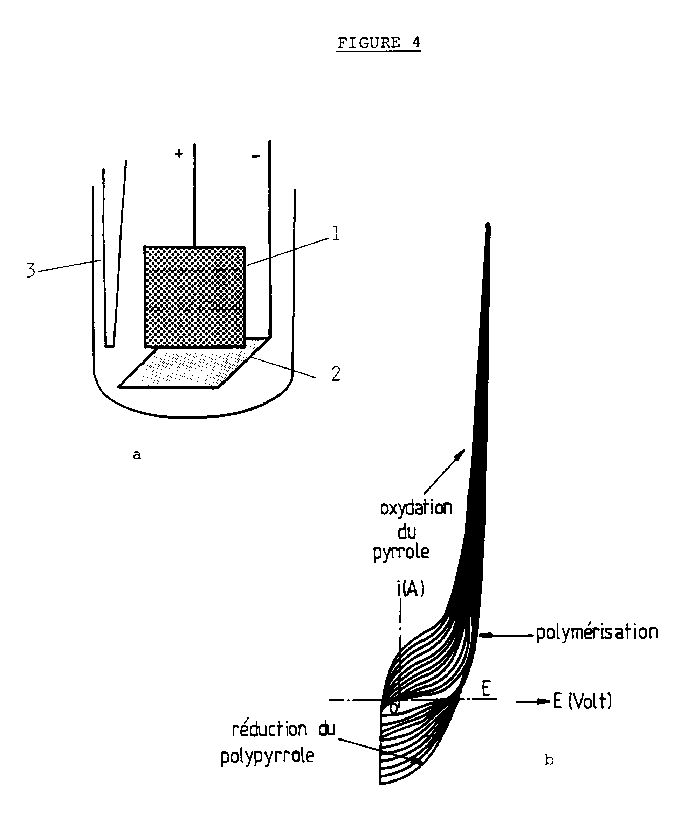

Les noyaux pyrroles oxydés sont capables de se polymériser pour former un polymère insoluble, le polypyrrole. Une cellule d'électropolymérisation est schématisée à la figure 4a : cette cellule comprend une électrode de travail (1), une contre-électrode (2), et une électrode de référence (3).

Si l'oxydation est réalisée par voie électrochimique, la synthèse du polypyrrole n'aura lieu que sur l'électrode de travail. Ceci permet donc une synthèse très localisée d'un polymère. Un oligonucléotide portant au bout d'un bras un noyau pyrrole peut donc être inséré dans le polymère simplement par copolymérisation des pyrroles. On obtient ainsi le polymère désiré (composé 6, figure 3).

Le polymère formé (polypyrrole) étant conducteur, ces réactions peuvent être poursuivies et plusieurs cycles de synthèse peuvent être réalisés (il y a seulement une variation de résistance de l'électrode à chaque cycle).

### B - Méthode

La polymérisation est conduite sur une électrode de platine de 60 mm² dans une solution contenant 10-² M de pyrrole, 5.10-⁷ M de pyrrole substitué, oligonucléotide porteur d'un groupe pyrrole en 5' (Oligo-1-pyr) et 0,1 M de LiClO₄ (dopant).

L'oligonucléotide portant le pyrrole en 5' (composé n° 5, Oligo-1-pyr) a été synthétisé selon la méthode décrite ci-dessus à l'exemple 1, et purifié par HPLC sur phase inverse. Un oligonucléotide de même séquence (Oligo-1) ne portant pas de pyrrole a servi de contrôle négatif.

Ces deux produits ont été marqués en 5' par du ³²P afin de suivre plus facilement les réactions de copolymérisation.

Les réactions d'oxydation du monomère et de réduction du polymère sont assurées par une variation cyclique du potentiel entre -0,4 et +0,9 V/ECS. La figure 4b représente les courbes de voltampérométrie cyclique (intensité en fonction du potentiel) au cours de 12 cycles de polymérisation.

L'intégration du courant par rapport au temps (quantité d'électrons consommée) permet une évaluation de la masse de polymère formé sur la surface de l'électrode et donc de l'épaisseur du film (de l'ordre de 0,2 µm pour 5.10⁻²C).

### C - Résultats

### * Stabilité d'un oligonucléotide dans les conditions d'électropolymérisation.

Le contrôle par HPLC de l'oligonucléotide en solution soumis à l'électropolymérisation ne montre aucune dégradation de celui-ci.

### * Migration propre d'un oligonucléotide soumis à un potentiel.

Un acide nucléique est une molécule polyanionique capable de migrer dans un champ électrique mais, de par la présence des ions perchlorate dans le milieu, au; cune migration n'est observée. D'autre part, aucune adsorption des oligonucléotides sur un polypyrrole préformé n'est mesurable.

### * Spécificité et du taux d'incorporation des oligonucléotides lors de la copolymérisation.

1 - La polymérisation du pyrrole est conduite en présence de l'oligonucléotide 1 non modifié oligo-1 (TGT ACC TGA ATC GTC CGC CAT).
   Oligo-1 : 10⁻⁹ M dans le milieu réactionnel
   Oligo-1 sur support : 4.10⁻¹² mol, soit 0,4% d'incorporation non spécifique.
2 - La polymérisation est conduite en présence de l'oligonucléotide modifié Oligo-1-pyr (P TGT ACC TGA ATC GTC CGC CAT)
   Oligo-1-pyr : 10⁻⁹ M dans le milieu réactionnel
   Oligo-1-pyr sur support : 7,2.10⁻¹² mol, soit 0,72% d'incorporation.
   44% des oligonucléotides-pyrroles détectés sur le support sont effectivement fixés par le groupe pyrrole. Cependant, en ajoutant 0,2 M de thymidine 5' phosphate dans la solution d'électropolymérisation, la spécificité d'accrochage s'élève alors à 80%, par diminution de la fixation de l'oligonucléotide non modifié.

### * Réactivité électrochimique de l'oligonucléotide-pyrrole.

La solution de départ contient 1 oligonucléotide-pyrrole pour 20 000 monomères pyrrole. Par le calcul de la masse du polymère formé et par la quantité d'oligonucléotide fixé, on estime que le polymère comprend 1 oligonucléotide-pyrrole pour 60 000 maillons pyrrole.

L'oligonucléotide-pyrrole s'incorpore donc 3 fois moins qu'un pyrrole libre, ce qui constitue un taux d'incorporation tout-à-fait satisfaisant.

### * Densité de fixation.

Dans les conditions expérimentales exposées ci-dessus, 5,3 pmoles/cm2 d'oligonucléotides sont fixées.

La proportion d'oligonucléotide intégré dans le polymère (1/60 000) peut être facilement améliorée par augmentation du rapport [oligonucléotide-pyrrole/pyrrole monomère] dans le milieu réactionnel. Ceci peut être réalisé de trois différentes façons
- Augmentation de la quantité d'oligonucléotide ;
- Diminution de la concentration de pyrrole libre ;
- Diminution du volume réactionnel.

### EXEMPLE 3 : PROPRIETES DES COPOLYMERES OLIGONUCLEOTIDES-POLYPYRROLE CONFORMES A L'INVENTION : UTILISATION COMME SUPPORT D'HYBRIDATION D'ACIDES NUCLEIQUES.

Un support polypyrrole portant l'oligonucléotide Oligo-1 a été synthétisé selon la méthode décrite à l'exemple 2. L'électropolymérisation a été conduite jusqu'aux charges de 5.10⁻² C pour obtenir un support de 0,2 µm d'épaisseur, et de 15.10⁻² C pour obtenir un support de 0,6 µm d'épaisseur. Les réactions d'hybridation sont effectuées dans un tampon phosphate 20 mM pH 7,4, NaCl 300 mM, SDS 0,5%. Les lavages sont réalisés dans le même tampon mais dilué 4 fois. Toutes ces réactions sont effectuées à température ambiante.

### Résultats

L'accessibilité des oligonucléotides greffés a été vérifiée par leur capacité d'hybridation avec d'un oligonucléotide complémentaire marqué au ³²P se trouvant dans le milieu liquide environnant.

### a) Hybridation

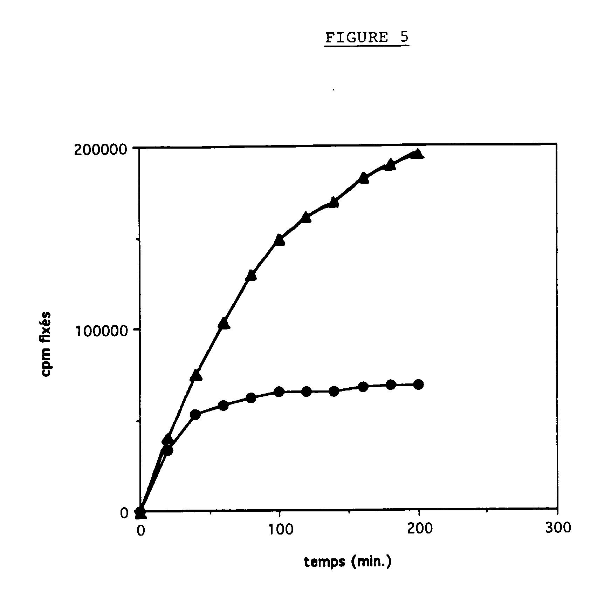

La cinétique d'hybridation des supports de différentes épaisseurs est comparable, et la capacité totale d'hybridation est proportionnelle à l'épaisseur du support, comme le montre la figure 5, qui représente en abscisse le temps d'hybridation (en minutes), et en ordonnée la quantité d'oligonucléotide complémentaire marqué au ³²P fixé au support (en cpm), pour deux épaisseurs différentes : (●) = support de 0,2 µm d'épaisseur ; (▲) = support de 0,6 µm d'épaisseur.

### b) Dénaturation

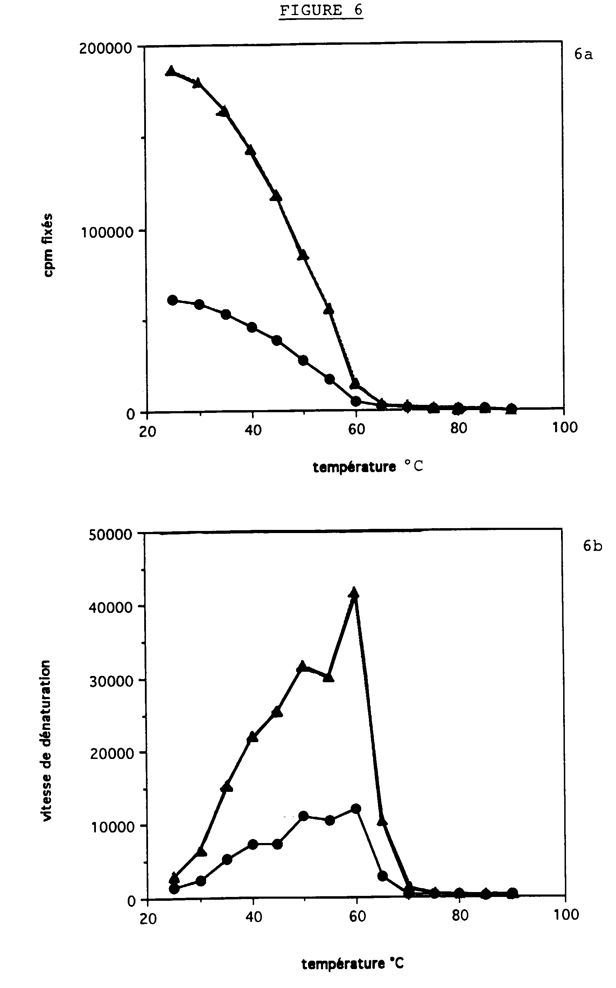

Il est possible de suivre la dénaturation des duplex de façon continue, ce qui montre la réversibilité du phénomène d'hybridation. Les figures 6a) et 6b) illustrent respectivement la quantité d'oligonucléotides restant sur l'électrode et la vitesse de dénaturation en fonction de la température de lavage (pour une variation de température de 1°C par minute) sur les supports d'épaisseur différente : (●) : 0,2 µm ; (▲) = 0,6 µm.

D'autre part, il a été vérifié que le support oligonucléotide-polypyrrole n'est pas affecté par des cycles de dénaturation/renaturation.

Dans les conditions expérimentales mises en oeuvre, la vitesse maximale de dénaturation est atteinte à 60°C environ, ce qui correspond au point de fusion théorique de l'oligonucléotide (61,5°C).

### EXEMPLE N° 4 : SYNTHESE IN SITU D'OLIGONUCLEOTIDES SUR SUPPORT POLYPYRROLE

### I. - FIXATION DU 1er NUCLEOTIDE

### 1ère METHODE

### Préparation du composé n° 13

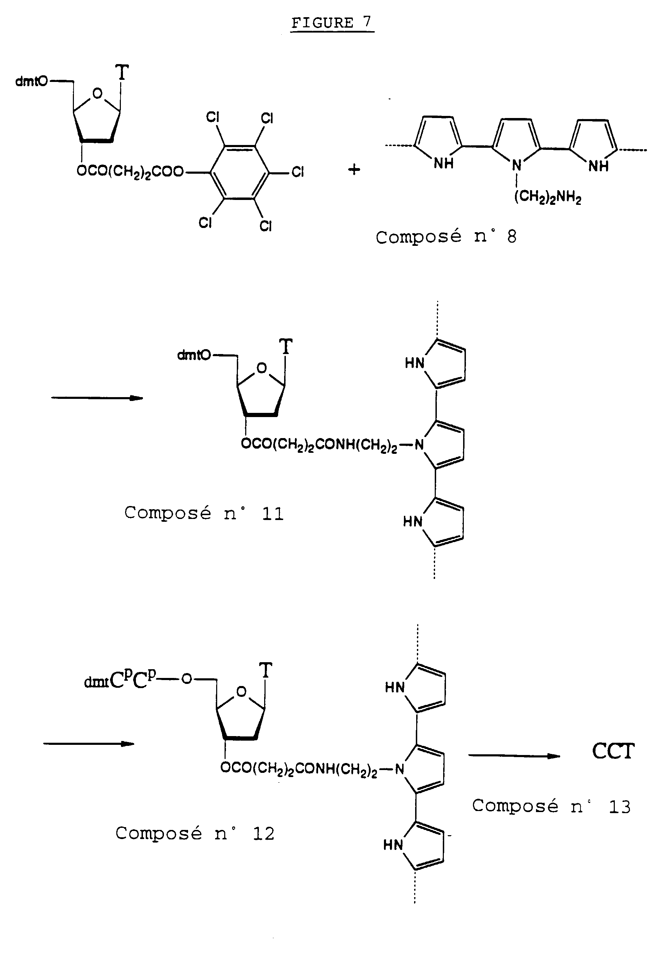

Le schéma réactionnel est illustré par la figure 7.

Le support (composé n°8, figure 7) est réalisé par électropolymérisation d'une solution de pyrrole et d'aminoéthylpyrrole (10⁻² M/10⁻³ M) en présence de LiClO₄ 0,1 M dans l'acétonitrile. L'électropolymérisation se fait par balayage de -0,3 V à +0,85 V par rapport à Ag/Ag+ 10⁻² M sur une électrode de platine de 60 mm².

### - Préparation du composé n° 11 (figure 7)

Le composé n°8 est lavé par de l'acétonitrile anhydre (2 x 5 ml) puis par de la triéthylamine dans l'acétonitrile (500 µl / 5 ml).

10 mg de nucléoside activé (composé n° 2) sont séchés par coévaporation dans l'acétonitrile anhydre, repris par 500 µl d'acétonitrile anhydre et ajoutés au support dans un flacon hermétiquement bouché. L'ensemble est placé sous agitation mécanique douce pendant 24 heures. Le support est retiré et lavé par de l'acétonitrile puis du dichlorométhane jusqu'à disparition de la couleur du trityle dans les solvants de lavage.

Les fonctions amine du support n'ayant pas réagi avec le nucléoside doivent être bloquées. Ceci a été effectué par "capping" par un mélange d'anhydride acétique-N-méthylimidazole dans la pyridine. La réaction est laissée 6 heures. Le support fonctionnalisé (composé n° 11) est ensuite lavé intensivement par 3 x 10ml de pyridine, 3 x 10ml d'acétonitrile et 3 x 10ml de dichlorométhane successivement.

### 2ème METHODE

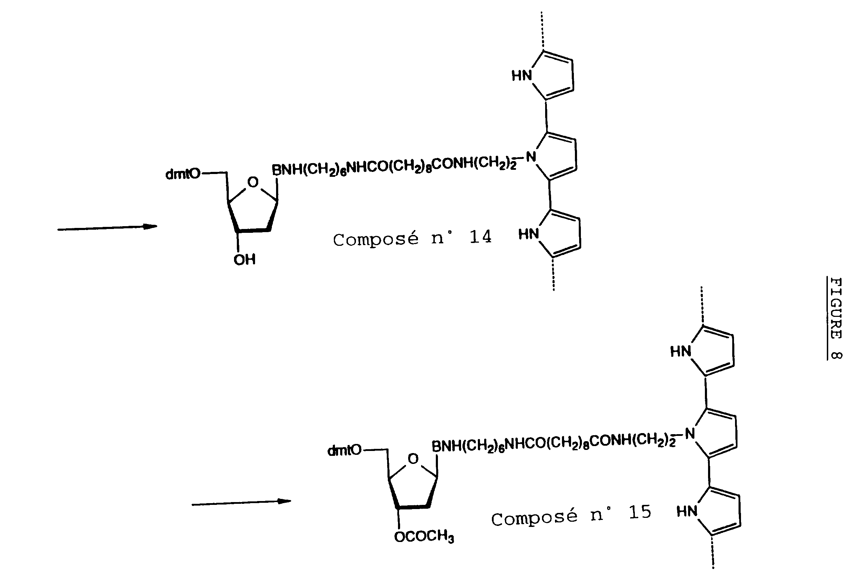

Le schéma réactionnel est illustré à la figure 8.

### - Préparation du composé n° 14 (figure 8)

Le polypyrrole aminé (composé n° 8) est lavé par de l'acétonitrile anhydre (2 x 5 ml) puis par de la triéthylamine dans l'acétonitrile (500 µl/5 ml). Le nucléoside activé (composé n° 2) (20 mg) est séché par coévaporation dans l'acétonitrile anhydre, repris par 1 ml d'acétonitrile anhydre et ajouté au support (composé n°8) dans un flacon hermétiquement bouché. L'ensemble est placé sous agitation mécanique douce pendant 24 heures. Le support greffé (composé n° 14) est lavé par de l'acétonitrile puis du dichlorométhane jusqu'à disparition de la couleur du trityle dans les solvants de lavage lors de leur acidification.

### - Préparation du composé n° 15 (figure 8)

Les fonctions alcool secondaire apportées par le nucléoside ainsi que les fonctions amine du support n'ayant pas réagi doivent être masquées. Pour cela, on réalise un blocage par un mélange d'anhydride acétique/N-méthylimidazole dans la pyridine (1 ml) pendant 6 heures. Un lavage par de la pyridine (2 x 5 ml), de l'acétronitrile (2 x 5 ml) et du dichlorométhane (2 x 5 ml) permet d'obtenir le composé n° 15.

### II. - ELONGATION DE L'OLIGONUCLEOTIDE

### - Préparation du composé n° 12 (figure 7)

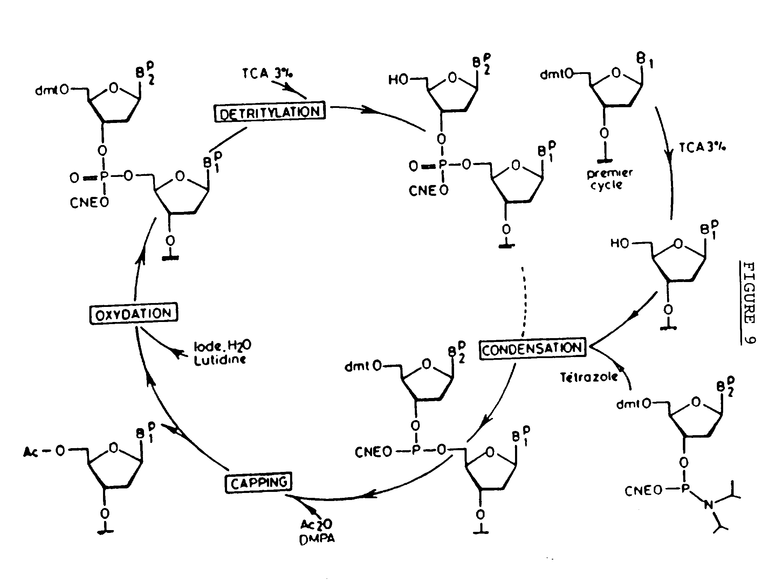

- Le trimère d(CCT) a été préparé sur électrode de platine recouverte de polypyrrole par deux méthodes :
. Synthèse avec déprotection chimique, suivant le cycle habituel de la synthèse phosphoramidite,
. Synthèse avec détritylation électrochimique.
   **a) Synthèse chimique**
      On effectue, autant de fois que nécessaire, les étapes suivantes, chacune correspondant à la fixation d'un nucléotide ; ces étapes sont représentées à la figure 9 :
      - Détritylation du support par 4 x 500 µl d'acide trichloroacétique à 2% dans le dichlorométhane ;
      - Rinçage par de l'acétonitrile pour enlever le réactif (5 x 1 ml) ;
      - Lavage par de l'acétonitrile anhydre pour synthèse d'ADN (3 x 1 ml) ;
      - Ajout de 250 µl de phosphoramidite 0,1 M et 250 µl de tétrazole 0,5 M ;
      - Couplage (2 mn) et élimination de la solution nucléosidique ;
      - Rinçage par de l'acétonitrile (5 x 1 ml) ;
      - Capping anhydride acétique/méthylimidazole (500 µl, 1mn) ;
      - Rinçage par de l'acétonitrile (2 x 1 ml) ;
      - Oxydation par iode/lutidine 1 mn (500 µl, 1 mn) ;
      - Rinçage par 5 x 1 ml d'acétonitrile ;
      - Détritylation, et début d'un nouveau cycle, etc ...
La mesure des trityles donne respectivement à l'issue de chaque cycle : 0,090 DO/2 ml (dT), 0,095 DO/2 ml (dCT) et 0,087 DO/2 ml (dCCT).

### b) Synthèse avec déprotection électrochimique

Les étapes de synthèse sont les mêmes que pour la synthèse chimique ci-dessus, mais la détritylation est réalisée par application d'un potentiel de 1,2 V pendant 5 mn.

La détritylation n'est pas quantifiable, car le cation trityle formé est capté par l'anode, ce qui le soustrait à la mesure. Le cycle de couplage a cependant été réalisé.

### - Préparation du composé n° 13 (figure 7)

La coupure du support et l'élimination des groupements protecteurs sont faites par 2 ml d'ammoniaque dans un tube en verre fermé par un bouchon à vis la réaction est effectuée pendant 48 heures à température ambiante.

Les témoins préparés sur colonne de silice sont déprotégés par 4 x 250 µl d'ammoniaque pour les décrocher du support (t = 4 x 1/2 h). La solution ammoniacale est ensuite laissée 48 heures à température ambiante. Les solutions sont évaporées et analysées par chromatographie en phase inverse sur une colonne C4, 5 µm de 25 cm. On applique un gradient de 0 à 30% de B (Acétate de triéthylammonium 25 mM, pH 7 et acétonitrile 50%) dans A (Acétate de triéthylammonium 25 mM, pH 7) en 30 min.

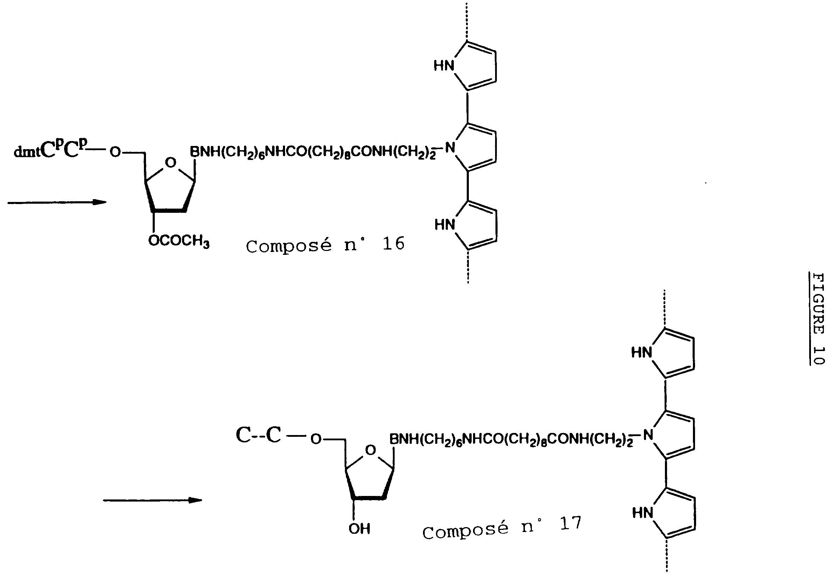

### - Préparation du composé n° 16 (figure 10)

Le composé n° 16 est synthétisé suivant le même protocole que le composé n° 12 avec des résultats semblables pour la détritylation. Ceci montre que la nature du bras espaceur influe peu sur la synthèse chimique.

### - Préparation du composé n° 17 (figure 10)

Le composé n° 16 est déprotégé 48 heures à température ambiante dans l'ammoniaque à 28% dans un flacon hermétiquement bouché. Le groupement diméthoxytrityle est ensuite coupé par l'acide trichloroacétique à 3% (3 x 3 ml) et mesuré pour vérifier que l'oligonucléotide est toujours sur le support.

### EXEMPLE N° 5 : COPOLYMERISATION D'OLIGONUCLEOTIDES-PYRROLE SUR DES MICROELECTRODES

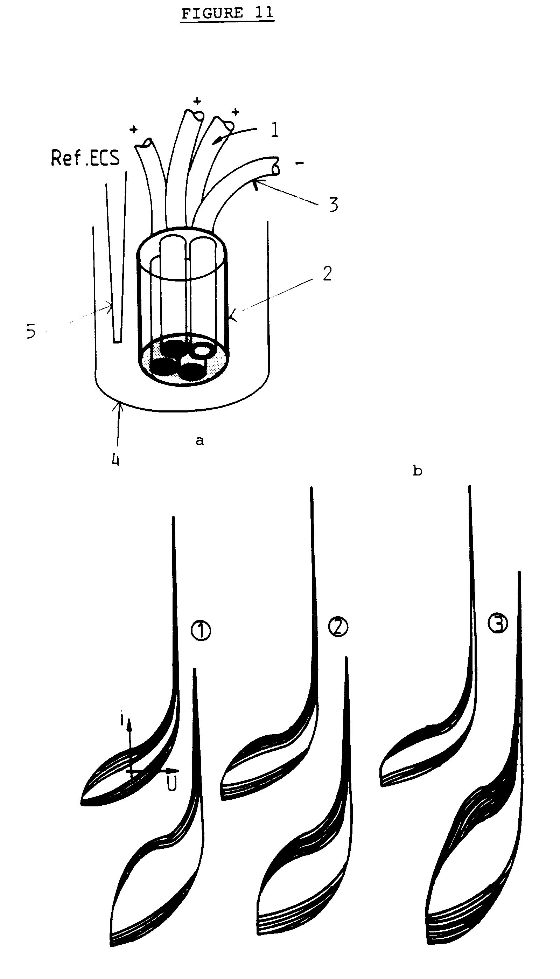

Une matrice de quatre électrodes représentée à la figure 11a est réalisée par inclusion de quatre fils de platine (1) (diamètre 0,6 mm) dans un cylindre de verre (2) (diamètre 5 mm x 10 mm de hauteur). Une des électrodes est utilisée comme contre-électrode (3). Ce système de matrice permet de fixer des oligonucléotides différents sur chaque point de la matrice.

La matrice d'électrodes est plongée dans un récipient (4), où est effectuée la réaction, et où est également immergée une électrode de référence (5).

Les 3 électrodes de travail sont successivement recouvertes électrochimiquement par un copolymère composé de pyrrole et d'oligonucléotides capables de détecter par hybridation une mutation du codon 61 du gène ras H humain. Ces 3 oligonucléotides portant en 5' un groupe pyrrole sont les suivants :

- oligo normal :    5' Pyr TCCTCCTGGCCGG 3'
- oligo muté A :    5' Pyr TCCTCCAGGCCGG 3'
- oligo muté C :    5' Pyr TCCTCCCGGCCGG 3'

Chaque oligonucléotide est copolymérisé successivement sur chaque électrode dans les conditions décrites dans l'exemple 2, mais dans un volume réactionnel de 300 µl au lieu de 3 ml.

Les voltampérogrammes obtenus sont représentés sur la figure llb : ((1) polymérisation sur la première électrode ; (2) polymérisation sur la deuxième électrode ; (3) polymérisation sur la troisième électrode) Ces voltampérogrammes sont très réguliers et très reproductibles aussi bien à charge réduite (2 à 4.10⁻⁴ C, courbes du haut) qu'à forte charge (1 à 1,3.10⁻³ C, courbes du bas). Dans ces conditions, 6.10⁻¹⁴ moles d'oligonucléotide sont fixées sur 0,3 mm² (soit 18 pmoles/cm²) pour une épaisseur de film de 0,1 µm (charge de 10⁻⁴ C).

### - Détection d'une mutation pontuelle d'un acide nucléique par hybridation sur une matrice 3 points.

Trois fragments d'acide nucléique d'une longueur de 51 nucléotides sont utilisés afin de simuler les mutations ras H naturelles recherchées.

Ces trois acides nucléiques ont pour séquence :

- ras H normal :
5' CTGTTGGACATCCTGGATGCCGGCCAGGAGGAGTACAGCGCCATGCGCGAC 3'
- ras H muté T :
5' CTGTTGGACATCCTGGATGCCGGCCTGGAGGAGTACAGCGCCATGCGCGAC 3'
- ras H muté G :
5' CTGTTGGACATCCTGGATGCCGGCCGGGAGGAGTACAGCGCCATGCGCGAC 3'

Ils sont spécifiquement reconnus par hybridation avec les sondes fixées sur la matrice ; respectivement oligo normal, oligo muté A, oligo muté C.

La réaction d'hybridation est réalisée à 25°C durant 1 heure, dans un tampon phosphate 20 mM, pH 7,4, NaCl 300 mM, SDS 0,5% contenant 0,1 pmole d'acide nucléique à détecter, marqué en 5' par du 32P. La matrice est ensuite lavée dans le même tampon à 35°C. La détection est réalisée par autoradiographie de la matrice sur un film photo. Dans ces conditions, l'hybridation de l'acide nucléique cible n'a lieu que sur l'électrode portant l'oligonucléotide de séquence strictement complémentaire ; aucune hybridation croisée n'est décelable.

La détection spécifique d'une mutation ponctuelle est donc possible grâce à cette matrice.

### EXEMPLE 6 : UTILISATION D'ULTRA-MICROELECTRODES.

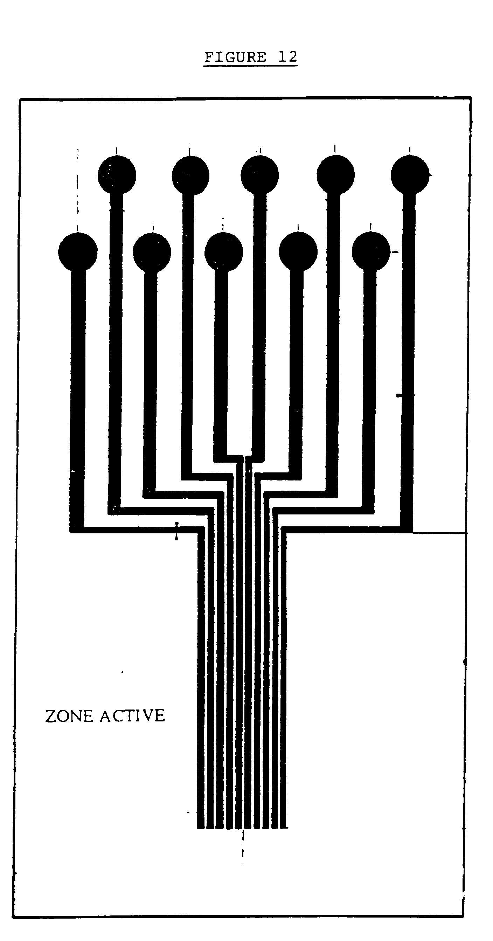

Un système représenté à la figure 12 est composé de 10 électrodes d'or déposées sur une plaquette de verre ; la largeur des électrodes peut varier de 10 à 100 µm et la longueur de la zone active (zone plongée dans la solution) est de l'ordre de 2 mm. Un autre système a été fabriqué par dépôt sélectif d'or sur un substrat isolant d'oxyde de silicium, puis isolation des connexions. Des matrices constituées d'électrodes carrées de 25 à 200 µm de côté sont ainsi obtenues.

Dans les deux cas, la copolymérisation de pyrrole et d'oligonucléotide-pyrrole peut être effectuée sur chaque électrode, selon le procédé décrit dans l'exemple 5 et les films de polypyrrole obtenus sont de bonne qualité et leur épaisseur peut être parfaitement maitrisée, comme décrit à l'exemple 2.

### EXEMPLE 7 : SYNTHESE IN SITU D'OLIGONUCLEOTIDES : DEPROTECTION ELECTROCHIMIQUE DE 5'-TTCTGAGG-3'

La synthèse a été conduite sur un support de polypyrrole aminé (composé n° 8), portant un bras clivable pour les besoins de l'analyse ultérieure de l'oligonucléotide formé.

### MODE OPERATOIRE :

### - Synthèse de 5'-TTCTGAGG-3' avec une étape de déprotection électrochimique de 5'-TTCTGAGG-3'

L'amidite de thymidine introduit en position (5) à partir de l'extrémité 3' de l'oligonucléotide est déprotégé par application d'un potentiel de +1,1 V pendant 15 mn lorsque le groupement protecteur est le thiopixyle, ou de -1,3 V pendant 15 mn lorsque le groupement protecteur est le groupement p-nitro-benzoyle. Les autres nucléosides sont introduits sous forme d'amidites tritylés, et sont déprotégés chimiquement, par détritylation par l'acide trichloroacétique.

### 1) Protection par un groupement p-nitrobenzoyle :

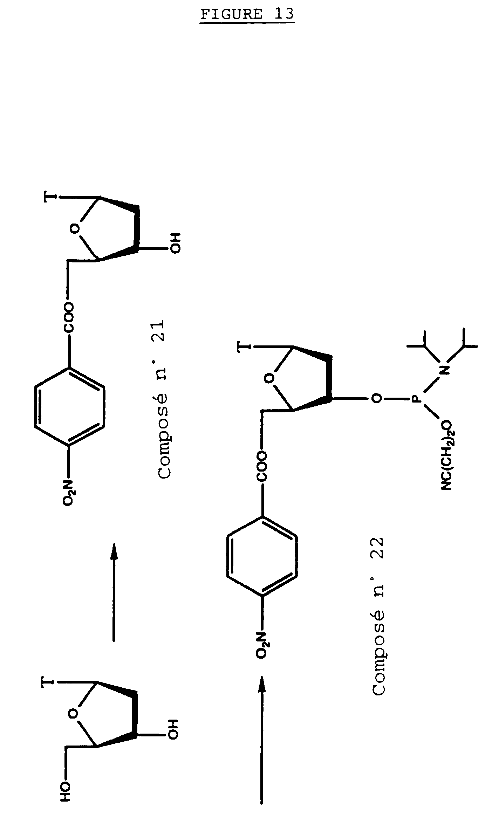

Les étapes de préparation du nucléoside protégé sont illustrées par la figure 13.

### - Synthèse de la p-nitrobenzoylthymidine (composé n° 21 : figure 13)

La thymidine (2,42 g ; 10 mmoles) est séchée par coévaporation dans la pyridine, puis reprise par 200 ml de pyridine anhydre et refroidie à 4°C. On ajoute le chlorure de p-nitrobenzoyle (2,04 g ; 11 mmoles). On laisse remonter la température et on laisse la réaction la nuit à température ambiante. La réaction est arrêtée par 5 ml de bicarbonate de sodium saturé. Le mélange réactionnel est concentré puis repris par 500 ml de chloroforme. La solution organique obtenue est lavée par 2 x 500 ml de NaHCO₃ 0,5 M puis 250 ml de NaCl saturé. Les phases aqueuses sont contre-extraites par 100 ml de CHCl₃. Les phases organiques sont évaporées. Le produit pur est obtenu par chromatographie sur colonne de silice. Il est élué par 5 % de méthanol dans le chloroforme. R = 58 %.

### - Synthèse de l'amidite de la p-nitrobenzoylthymidine (composé n° 22 : figure 13)

Le composé n° 21 (1.96 g ; 5 mmoles) et le tétrazolate de diisopropylammonium (428 mg ; 2.5 mmole) sont séchés par coévaporation à partir d'un solvant dichlorométhane/acétonitrile anhydre. Ces réactifs sont repris par 25 ml de dichlorométhane anhydre et on ajoute la bis-diisopropylaminocyanoéthoxyphosphine (1.8 g ; 6 mmoles). Après 2 h de réaction (à l'abri de l'oxygène et de l'humidité) le mélange réactionnel est dilué par 250 ml de dichlorométhane et lavé successivement par 2 X 250 ml de bicarbonate de sodium 0,5 M et 250 ml de chlorure de sodium saturé. La phase organique est évaporée. Le résidu est repris par 10 ml de dichlorométhane. Le produit (composé n° 22) est obtenu par précipitation dans l'hexane, puis séché sous vide et stocké sous argon. R = 84 %.

### 2) Protection par un groupement thiopixyle

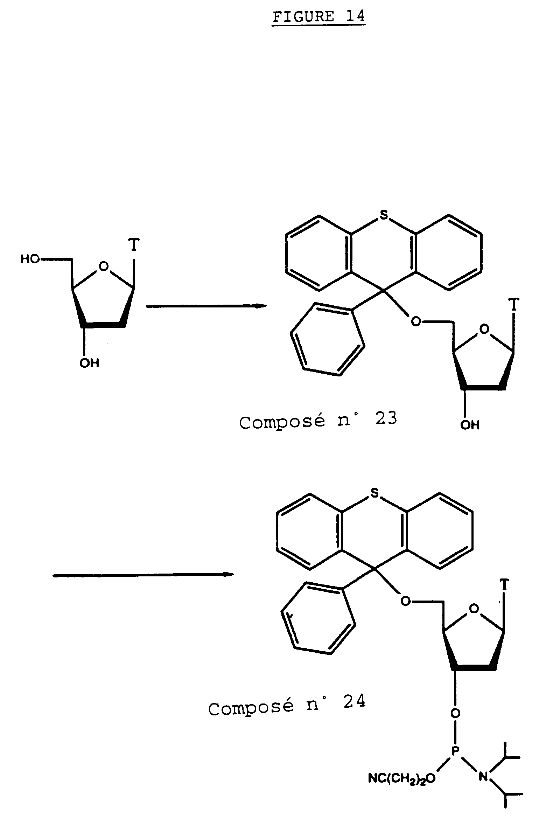

Les étapes de préparation du nucléoside protégé sont illustrées par la figure 14.

### - Synthèse de la thiopixylthymidine (composé n° 23 : figure 14)

La thymidine (2.42 g ; 10 mmoles) est séchée par coévaporation dans la pyridine, reprise par 100 ml de pyridine anhydre, refroidie à 4°C, et mise en réaction avec le chlorure de thiopixyle (3.4 g ; 11 mmoles). Après remontée progressive de la température jusqu'à la température ambiante, on laisse la réaction s'effectuée penant une nuit (8 à 12 environ) . La réaction est stoppée par 10 ml de NaHCO₃. Le solvant est évaporé, et le résidu est repris par 250 ml de dichlorométhane. La solution organique obtenue est extraite par 2 x 250 ml de NaHCO₃ saturé puis par 250 ml d'eau distillée. Les phases aqueuses sont contre-extraites par 100 ml de chloroforme. La phase organique est évaporée. Le produit est purifié sur colonne de silice avec 0,5 % de TEA (triéthylamine) dans les solvants. Il est enfin élué par 5 % de méthanol dans le dichlorométhane (+ 0,5 % TEA). R = 52%.

### - Synthèse de l'amidite de la thiopixylthymidine (composé n° 24, figure 14)

Le composé n° 24 est préparé à partir du composé n° 23 (5 mmoles) suivant le même mode opératoire que le composé n° 22. R = 78 %.

### 3) Fixation du premier nucléoside

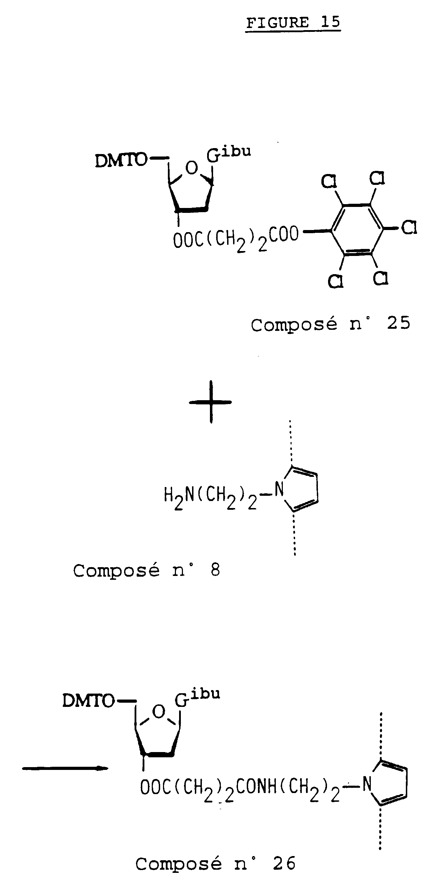

Les étapes de cette fixation sont illustrées à la figure 15.

La synthèse de cet oligonucléotide se fait sur un ruban de platine (3 x 10 mm) sur lequel on a copolymérisé un mélange de pyrrole et d'aminoéthyl pyrrole (9:1).

Le premier nucléoside (position 1- extrémité 3') est couplé sur l'amino éthyl pyrrole (composé n° 8) suivant les méthodes décrites pour la fonctionnalisation des supports de silice [K. MIYOSHI et al, Nucleic Acids Res. ; 8, (22), 5473-5489 (1989)], à partir d'un ester activé N-isobutyryl 2'-désoxyguanosine (composé n° 25 : (figure 13a).

Le composé n° 26 (10 mg) est dissous dans 500 µl d'acétonitrile. On ajoute l'électrode de platine recouverte de polypyrrole fonctionnalisé, et 1 µl de triéthylamine. La réaction est agitée mécaniquement 20 heures à température ambiante. L'électrode greffée (composé n° 26) est retirée, et lavée abondamment par de l'acétonitrile puis par du dichlorométhane.

Les fonctions amine n'ayant pas réagi sont bloquées à l'anhydride acétique (10 % dans 500 µl de pyridine) pendant 6 heures. L'électrode greffée est lavée à la pyridine et au méthanol puis séchée.

### 4) Synthèse de l'oligonucléotide dTTCTGAGG

L'électrode greffée est placée dans une colonne OPC® vidée (APPLIED BIOSYSTEMS). Le remplissage est terminé par des copeaux de téflon afin de minimiser le volume résiduel. Les nucléosides en position 2, 3, et 4 sont ajoutés suivant les prescriptions du fabricant (APPLIED BIOSYSTEMS) pour le "cycle 1 µmole" sur le synthétiseur 381A. La déprotection chimique du groupe diméthoxytrityle entre chaque étape est réalisée par le TCA dans le dichlorométhane dans les conditions recommandées par le fabricant.

L'amidite en position 5 (composé n° 22 ou composé n° 24) est couplé suivant la même méthode que les amidites normaux avec un temps de couplage de 1 mn. On procède à l'oxydation de la liaison phosphite triester créée, et au capping suivant le mode opératoire classique. L'électrode est sortie, de la colonne et on procède à la déprotection électrochimique.

Si on utilise le composé n° 22 le groupement p-nitrobenzoyle est coupé en plongeant l'électrode dans l'électrolyte suivant : tétrabutylammonium perchlorate 0,1 M dans le méthanol, et en appliquant un potentiel de -1,3 V pendant 15 mn.

Si on utilise le composé n° 24 le thiopixyle est coupé en appliquant un potentiel de +1,1 V pendant 15 mn, l'électrolyte étant du tétrabutylammonium perchlorate 0,1 M dans l'acétonitrile.

Dans les deux cas, après coupure du groupement protecteur du nucléosite T en position 5', l'électrode est replacée dans la colonne avec les copeaux de téflon, et la synthèse est poursuivie en ajoutant successivement les amidites C (position 6), T (position 7), T (position 8).

Quand la synthèse est terminée l'oligonucléotide est coupé du support : l'électrode est traitée par 4 x 500 µl d'ammoniaque à 28 % en flacon bouché pendant 4 x 1/2 h. Les 4 fractions sont regroupées dans un flacon WHEATON, de 4 ml bouché, et laissées 16 h à 55°C pour déprotéger l'oligonucléotide.

Après coévaporation en présence de TEA, un aliquot (1/100ème) de l'oligonucléotide obtenu est marqué en 5' par du 32P en présence de polynucléotide kinase puis analysé par électrophorèse sur gel de polyacrylamide. L'analyse par électrophorèse sur gel d'acrylamide montre la présence du produit désiré (8 mère) ainsi que l'absence de l'oligonucléotide (5 mère) dont la présence indiquerait une mauvaise déprotection électrochimique de l'amidite de thymidine.

## Revendications

1. Copolymère, caractérisé en ce qu'il répond à la formule générale (I) suivante : 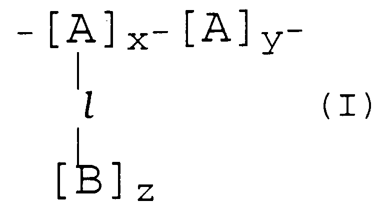 dans laquelle l'unité A représente un monomère d'un polymère conducteur électronique, l'unité B représente un nucléotide, un oligonucléotide ou un de leurs analogues, x, y et z représentent des nombres entiers égaux ou supérieurs à 1, ou y peut être égal à 0, et *l* représente une liaison covalente, ou un bras espaceur.

2. Copolymère selon la revendication 1, caractérisé en ce que A représente une unité monomère d'un PCE choisi dans le groupe comprenant le polyacétylène, la polyazine, le poly(p-phénylène), le poly(p-phénylène vinylène), le polypyrène, le polypyrrole, le polythiophène, le polyfuranne, le polysélénophène, la polypyridazine, le polycarbazole, la polyaniline.

3. Copolymère selon la revendication 2, caractérisé en ce que A est une unité pyrrole.

4. Copolymère selon l'une quelconque des revendications 1 à 3, caractérisé en ce que le rapport x/y est compris entre 1/5 et 1/100.000

5. Copolymère selon l'une quelconque des revendications 1 à 4, caractérisé en ce que *l* représente un bras espaceur répondant à la formule suivante :
-R₁-[(CH₂)ₙ-R₂]ₓ-[(CH₂)ₘ-R₃]_{y}-(CH₂)ₚ-
dans laquelle :
- n est un nombre entier de 1 à 10 ;
- m est égal à 0 ou est un nombre entier de 1 à 10 ;
- p est égal à 0 ou est un nombre entier de 1 à 10 ;
- x est égal à 0 ou est un nombre entier de 1 à 8 ;
- y est égal à 0 ou est un nombre entier de 1 à 8 ;
- R₁, R₂ et R₃ qui peuvent être identiques ou différents représentent :
CH2; O; S; NR'; CO; CH=CH ; NR'CO; CONR'; NHSO₂; 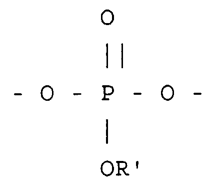 où R' représente un atome d'hydrogène ou une chaîne alkyle en C₁ à C₁₂.

6. Composé de formule générale (IV) : 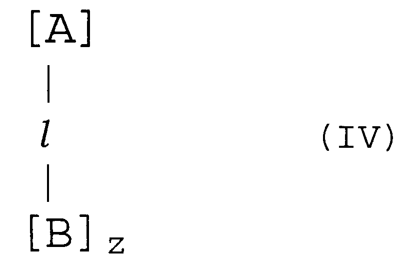 dans lequel A, B, z, et *l* sont tels que définis dans l'une quelconque des revendications 1 à 3, ou 5.

7. Procédé de préparation d'un copolymère de formule générale (I) selon une quelconque des revendications 1 à 5, caractérisé en ce qu'il comprend au moins les étapes suivantes :
- une première étape, au cours de laquelle on procède à la préparation d'un copolymère de formule générale (II) :
- [A*]ₓ- [A]_{y} - (II)
dans laquelle A, x et y sont tels que définis dans la revendication 1, et A* représente A fonctionnalisé.
- une deuxième étape au cours de laquelle on procède à la fixation, sur le polymère de formule (II) d'au moins un groupe de formule générale (III) :
*l**-[B]_{z} (III)
dans laquelle B et z sont tels que définis précédemment et *l** est un bras activé capable de se lier à A*.

8. Procédé selon la revendication 7, caractérisé en ce que l'étape de préparation du copolymère de formule générale (II) et/ou l'étape de fixation du groupe de formule générale (III) sont effectuées par réaction électrochimique.

9. Procédé de préparation d'un copolymère de formule générale (I) selon l'une quelconque des revendications 1 à 5, caractérisé en ce qu'il comprend au moins les étapes suivantes :
- une étape a) au cours de laquelle on procède à la préparation du composé de formule générale (IV) : 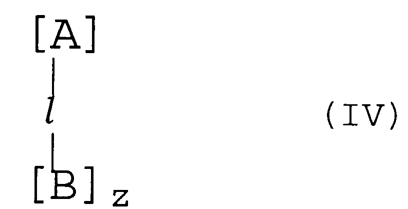 dans lequel A, B, z, et *l* sont tels que définis ci-dessus,
- une étape b) au cours de laquelle on procède à la copolymérisation du composé (IV) avec le monomère A.

10. Procédé selon la revendication 9, caractérisé en ce que l'étape b), est effectuée par copolymérisation électrochimique du composé (IV) avec les monomères A.

11. Procédé selon l'une quelconque des revendications 7 à 10, caractérisé en ce que l'on procède en outre à l'élongation de B_{z}, en plusieurs étapes successives, chacune de ces étapes étant constituée par la fixation d'un ou plusieurs unités B.

12. Procédé selon la revendication 11, caractérisé en ce que l'élongation de B_{z} fait intervenir une suite de réactions électrochimiques.

13. Procédé selon l'une quelconque des revendications 10 à 12, caractérisé en ce que l'élongation de B_{z} est effectuée en mettant en oeuvre au moins un dérivé de thymidine de formule : 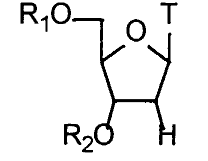 dans laquelle T représente la thymine, R1 représente un groupe p-nitrobenzoyle ou un groupe thiopixyle, R2 représente un atome d'hydrogène ou bien un groupe de formule : 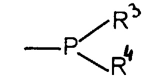 dans laquelle R3 représente un groupe diisopropylammonium, et R4 représente un groupe -O(CH₂)₂CN,
et fait intervenir au moins une réaction électrochimique.

14. Procédé selon une quelconque des revendications 8, 11, 12 ou 13, caractérisé en ce qu'il est mis en oeuvre à la surface d'une électrode.

15. Utilisation d'un polymère conducteur électronique comme support pour la fixation par liaison covalente, d'au moins un nucléotide, ou d'au moins un oligonucléotide.

16. Utilisation d'un copolymère selon l'une quelconque des revendication 1 à 5 comme support de synthèse polynucléotidique.

17. Utilisation d'un copolymère selon l'une quelconque des revendication 1 à 5 comme support d'hybridation d'acides nucléiques.

18. Electrode, caractérisée en ce que sa surface est constituée par un revêtement comprenant un copolymère de formule (I) selon l'une quelconque des revendications 1 à 5.

19. Dispositif utilisable pour des réactions de synthèse et d'hybridation d'acides nucléiques, caractérisé en ce qu'il comprend une ou plusieurs électrodes selon la revendication 18, lesquelles électrodes peuvent être identiques ou différentes.

20. Dispositif selon la revendication 19, caractérisé en ce qu'il comprend plusieurs électrodes, dont au moins deux portent chacune un groupe Bz différent.

## Patentansprüche

1. Copolymeres, dadurch **gekennzeichnet**, daß es der folgenden allgemeinen Formel (I) : 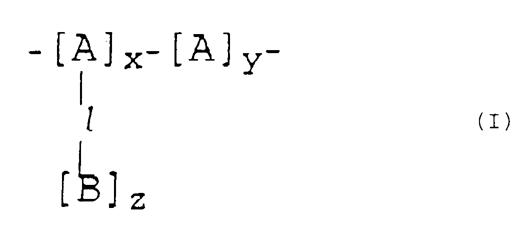 entspricht, worin die Einheit A ein Monomeres eines elektronisch leitenden Polymeren bezeichnet, die Einheit B ein Nucleotid, ein Oligonucleotid oder eines ihrer Analoga bezeichnet, x, y und z ganze Zahlen von gleich oder größer 1 bedeuten oder y 0 sein kann und *l* eine kovalente Bindung oder einen Spacer bezeichnet.

2. Copolymeres nach Anspruch 1, dadurch **gekennzeichnet**, daß A eine Monomereinheit eines PCEs, ausgewählt aus der Gruppe, umfassend Polyacetylen, Polyazin, Poly-(p-phenylen), Poly-(p-phenylenvinylen), Polypyren, Polypyrrol, Polythiophen, Polyfuran, Polyselenophen, Polypyridazin, Polycarbazol, Polyanilin bedeutet.

3. Copolymeres nach Anspruch 2, dadurch **gekennzeichnet**, daß A eine Pyrroleinheit ist.

4. Copolymeres nach einem der Ansprüche 1 bis 3, dadurch **gekennzeichnet**, daß das Verhältnis x/y zwischen 1/5 und 1/100.000 liegt.

5. Copolymeres nach einem der Ansprüche 1 bis 4, dadurch **gekennzeichnet**, daß *l* einen Spacer der folgenden Formel:
-R₁-[(CH₂)ₙ-R₂]ₓ-[(CH₂)ₘ-R₃]_{y}-(CH₂)ₚ-
bezeichnet, worin
- n eine ganze Zahl von 1 bis 10 bedeutet;
- m den Wert 0 hat oder eine ganze Zahl von 1 bis 10 ist;
- p den Wert 0 hat oder eine ganze Zahl von 1 bis 10 ist;
- x den Wert 0 hat oder eine ganze Zahl von 1 bis 8 ist;
- y den Wert 0 hat oder eine ganze Zahl von 1 bis 8 ist;
- R₁, R₂ und R₃, die gleich oder verschieden sein können, für:
CH₂; O; S; NR'; CO; CH=CH; NR'CO; CONR'; NHSO₂; 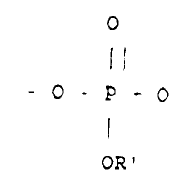
stehen, worin R' ein Wasserstoffatom oder eine C₁-C₁₂-Alkylenkette ist.

6. Verbindung der allgemeinen Formel (IV) : 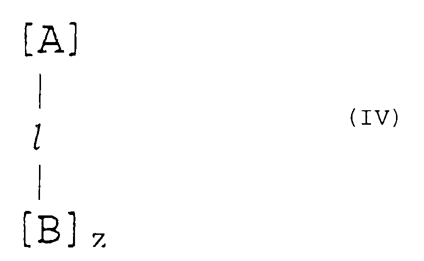 worin A, B, z und 1 wie in einem der Ansprüche 1 bis 3 oder 5 definiert sind.

7. Verfahren zur Herstellung eines Copolymeren der allgemeinen Formel (I) nach einem der Ansprüche 1 bis 5, dadurch **gekennzeichnet**, daß es mindestens die folgenden Stufen umfaßt:
eine erste Stufe, im Verlauf derer ein Copolymeres der allgemeinen Formel (II) :
- [A*]ₓ- [A]_{y} - (II)
hergestellt wird, worin A, x und y wie in Anspruch 1 definiert sind, und A* ein derivatisiertes A bedeutet,
- eine zweite Stufe, im Verlauf derer man an das Polymere der Formel (II) mindestens eine Gruppe der allgemeinen Formel (III)
*l**-[B]_{z} (III)
bindet, worin B und z wie vorstehend definiert sind, und l* ein aktivierter Spacer ist, der an A* binden kann.

8. Verfahren nach Anspruch 7, dadurch **gekennzeichnet**, daß die Stufe der Herstellung des Copolymeren der allgemeinen Formel (II) und/oder die Stufe der Bindung der Gruppe der allgemeinen Formel (III) durch elektrochemische Reaktion durchgeführt werden.

9. Verfahren zur Herstellung eines Copolymeren der allgemeinen Formel (I) nach einem der Ansprüche 1 bis 5, dadurch **gekennzeichnet**, daß es mindestens die folgenden Stufen umfaßt:
- eine Stufe a), im Verlauf derer man eine Verbindung der allgemeinen Formel (IV): 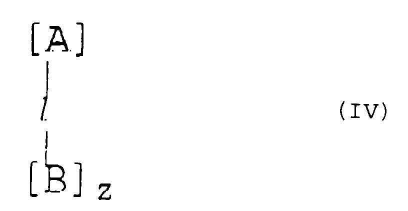 herstellt, worin A, B, z und l wie vorstehend definiert sind,
- eine Stufe b), im Verlauf derer man die Verbindung (IV) mit dem Monomeren A copolymerisiert.

10. Verfahren nach Anspruch 9, dadurch **gekennzeichnet**, daß die Stufe b) durch elektrochemische Copolymerisation der Verbindung (IV) mit den Monomeren A durchgeführt wird.

11. Verfahren nach einem der Ansprüche 7 bis 10, dadurch **gekennzeichnet**, daß man weiterhin B₂ in mehreren aufeinanderfolgenden Stufen verlängert, wobei jede dieser Stufen aus der Bindung einer oder mehrerer Einheiten B besteht.

12. Verfahren nach Anspruch 11, dadurch **gekennzeichnet**, daß die Verlängerung von B₂ über eine Folge von elektrochemischen Reaktionen erfolgt.

13. Verfahren nach einem der Ansprüche 10 bis 12, dadurch **gekennzeichnet**, daß die Verlängerung von B_{z} durchgeführt wird, indem mindestens ein Thymidinderivat der Formel: 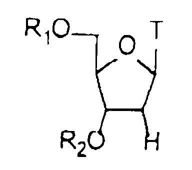 eingesetzt wird, worin T Thymin bedeutet, R₁ eine p-Nitrobenzoylgruppe oder eine Thiopixylgruppe bedeutet, R₂ ein Wasserstoffatom oder auch eine Gruppe der Formel: 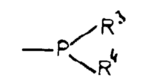 bedeutet, worin R₃ eine Diisopropylammoniumgruppe bedeutet und R₄ eine Gruppe
-O(CH₂)₂CN
bedeutet, und mindestens eine elektrochemische Reaktion als Zwischenreaktion erfolgt.

14. Verfahren nach einem der Ansprüche 8, 11, 12 oder 13, dadurch **gekennzeichnet**, daß es an der Oberfläche einer Elektrode durchgeführt wird.

15. Verwendung eines elektronisch leitenden Polymeren als Träger zur Fixierung über kovalente Bindung mindestens eines Nucleotids oder mindestens eines Oligonucleotids.

16. Verwendung eines Copolymeren nach einem der Ansprüche 1 bis 5 als Träger zur Polynucleotidsynthese.

17. Verwendung eines Copolymeren nach einem der Ansprüche 1 bis 5 als Träger zur Hybridisierung von Nucleinsäuren.

18. Elektrode, dadurch **gekennzeichnet**, daß ihre Oberfläche aus einem Überzug besteht, umfassend ein Copolymeres der Formel (I) nach einem der Ansprüche 1 bis 5.

19. Vorrichtung zur Verwertung für Synthese und Hybridisierungsreaktionen von Nucleinsäuren, dadurch **gekennzeichnet**, daß sie eine oder mehrere Elektrode(n) nach Anspruch 18 umfaßt, wobei die Elektroden gleich oder verschieden sein können.

20. Vorrichtung nach Anspruch 19, dadurch **gekennzeichnet**, daß sie mehrere Elektroden umfaßt, von denen mindestens zwei jeweils eine unterschiedliche B_{z}-Gruppe tragen.

## Claims

1. A copolymer which corresponds to the following general formula (I): 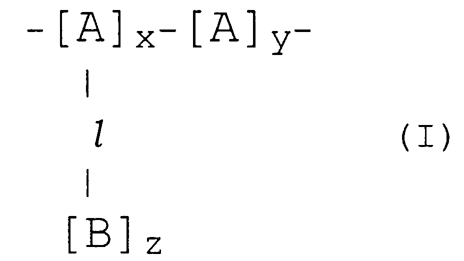 in which the unit A represents a monomer of an electrically conductive polymer, the unit B represents a nucleotide, an oligonucleotide or one of the analogues thereof, x, y and z represent integers equal to or greater than 1, or y may be equal to 0, and *l* represents a covalent bond or a spacer arm.

2. The copolymer as claimed in claim 1, wherein A represents a monomer unit of an ECP chosen from the group comprising polyacetylene, polyazine, poly(p-phenylene), poly(p-phenylene vinylene), polypyrene, polypyrrole, polythiophene, polyfuran, polyselenophene, polypyridazine, polycarbazole and polyaniline.

3. The copolymer as claimed in claim 2, wherein A is a pyrrole unit.

4. The copolymer as claimed in any one of claims 1 to 3, wherein the ratio x/y is between 1/5 and 1/100,000.

5. The copolymer as claimed in any one of claims 1 to 4, wherein *l* represents a spacer arm corresponding to the following formula:
-R₁-[(CH₂)ₙ-R₂]ₓ-[CH₂)ₘ-R₃]_{y}-(CH₂)ₚ-
in which:
- n is an integer from 1 to 10;
- m is equal to 0 or is an integer from 1 to 10;
- p is equal to 0 or is an integer from 1 to 10;
- x is equal to 0 or is an integer from 1 to 8;
- y is equal to 0 or is an integer from 1 to 8;
- R₁, R₂ and R₃, which may be identical or different, represent:
CH₂ ; 0 ; S; NR' ; CO; CH=CH; NR'CO; CONR'; NHSO₂; 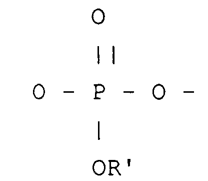
where R' represents a hydrogen atom or a C₁ to C₁₂ alkyl chain.

6. compound of general formula (IV) : 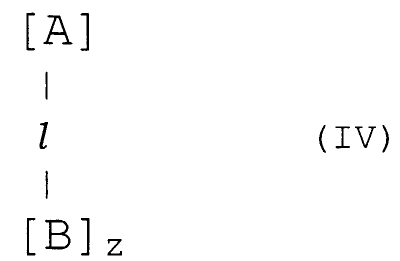 in which A, B, z, and *I* are as defined in any of claims 1 to 3 or 5.

7. A process for the preparation of a copolymer of general formula (I) as claimed in any one of claims 1 to 5, wherein it comprises at least the following steps:
- a first step, during which there is prepared a copolymer of general formula (II) :
- [A*]ₓ-[A]_{y} - (II)
in which A, x and y are as defined in claim 1, and A* represents functionalized A.
- a second step, during which there is bound, to the polymer of formula (II), at least one group of general formula (III):
*l**-[B]_{z} (III)
in which B and z are as defined above and *l** is an activated arm capable of binding to A*.

8. The process as claimed in claim 7, wherein the step for the preparation of the copolymer of general formula (II) and/or the step for the binding of the group of general formula (III) are carried out by electrochemical reaction.

9. A process for the preparation of a copolymer of general formula (I) as claimed in any one of claims 1 to 5, wherein it comprises at least the following steps:
- a step a) during which there is prepared the compound of general formula (IV): 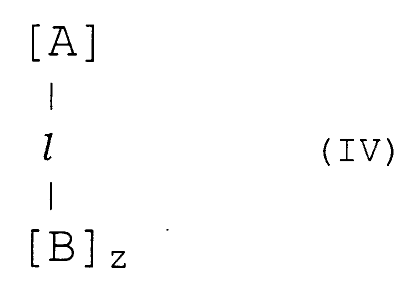 in which A, B, z and *l* are as defined above,
- a step b) during which there is copolymerized the compound (IV) with the monomer A.

10. The process as claimed in claim 9, wherein step b) is carried out by electrochemical copolymerization of the compound (IV) with the monomers A.

11. The process as claimed in any one of claims 7 to 10, wherein B_{z} is elongated in several successive steps, each of these steps consisting of the binding of one or more units B.

12. The process as claimed in claim 11, wherein the elongation of B_{z} involves a sequence of electrochemical reactions.

13. The process as claimed in any of claims 10 to 12 wherein the elongation of B_{z} involves the use of at least a thymidine derivative of formula : 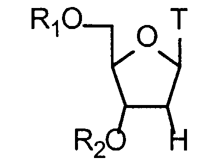 wherein T is thymine, R1 is a p-nitrobenzoyl or thiopixyl group, R2 is an hydrogen atom or a group of formula : 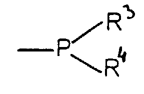 wherein R3 is a diisopropylammonium group and R4 is
O(CH₂)₂CN,
and involves at least an electrochemical reaction.

14. The process as claimed in any one of claims 8, 11, 12, or 13, which is carried out at the surface of an electrode.

15. Use of an electrically conductive polymer as a support for the binding, by covalent bonding, of at least one nucleotide, or of at least one oligonucleotide.

16. The use of a copolymer as claimed in any one of claims 1 to 5 as a polynucleotide synthesis support.

17. The use of a copolymer as claimed in any one of claims 1 to 5 as a hybridization support for nucleic acids.

18. An electrode, the surface of which consists of a coating comprising a copolymer of formula (I) as claimed in any one of claims 1 to 5.

19. A device which may be used for nucleic acid synthesis and hybridization reactions, which device comprises one or more electrodes as claimed in claim 18, which electrodes may be identical or different.

20. The device as claimed in claim 19, which comprises several electrodes, at least two of which each bear a different group B_{z}.
